# EUROPÄISCHE PATENTANMELDUNG

(11) **EP 2 308 537 A1**
(43) Veröffentlichungstag der Anmeldung: **13.04.2011**
(21) Anmeldenummer: 10185042.8
(22) Anmeldetag: 16.06.2003
(51) Int. Cl.: A61M 16/06

(54) **Maskenkissen für eine Atemmaske**

(30) Priorität: 14.06.2002 DE 10226587; 26.06.2002 DE 10228554
(62) Teilanmeldung aus: 03759964.4
(71) Anmelder: MAP Medizin-Technologie GmbH, 82152 Martinsried (DE)
(72) Erfinder: Lang, Bernd, 82166 Gräfelfing (DE); Biener, Achim, 85445 Aufkirchen (DE); Bechtel, Martin, 80339 München (DE)
(74) Vertreter: Vossius & Partner

(57) **Zusammenfassung**

Maskenkisseneinrichtung (1) für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe (3), die in Applikationsposition auf der Gesichtsfläche des Maskenanwenders aufsitzt, wobei in dem Maskenkissen wenigstens ein Aufnahmetaschewnabschnitt (22) ausgebildet ist und in diesem Aufnahmetaschenabschnitt ein Polsterkörper aus einem Gel- oder Schaummaterial vorgesehen ist.

## Beschreibung

### Gebiet der Erfindung

Die Erfindung betrifft ein Maskenkissen sowie ein Stirnpad für eine Atemmaske, eine Atemmaske an sich sowie ein Formwerkzeug und ein Verfahren zur Herstellung derselben. Die Erfindung betrifft insbesondere Maskenkissen bzw. eine mit einem derartigen Maskenkissen ausgestattete Atemmaske, durch welche in abdichtender Weise ein Atemmaskeninnenraum im Zusammenspiel mit der Gesichtsfläche eines Maskenanwenders derart gegenüber der Umgebung abdichtbar ist, dass in diesem Atemmaskeninnenraum zumindest phasenweise ein gegenüber dem Umgebungsdruck erhöhter Druck herrschen kann. Derartige Atemmasken finden insbesondere im Zusammenhang mit der medizinischen oder therapeutischen Verabreichung von atembaren Gasen sowie auch im technischen Bereich, zum Beispiel im Bereich der Atemschutztechnik, Anwendung. Die Erfindung befasst sich weiterhin auch mit einer Dichtungsstruktur und einem Verfahren zur Herstellung derselben im allgemeinen.

Üblicherweise wird bei diesen Atemmasken die Abdichtung zur Gesichtsfläche des Atemmaskenanwenders durch eine, um eine Maskenöffnung einwärtsgerichtet umlaufende und aus einem elastisch verformbaren Material gefertigte Dichtlippenstruktur erreicht.

Die mit derartigen Dichtlippen erreichte Dichtwirkung nimmt allgemein mit dem Anpressdruck der dichtlippe gegen die Gesichtsfläche zu. Im Falle hoher Anpressdrücke kann insbesondere die Langzeitanwendung derartiger Atemmasken Unannehmlichkeiten bereiten.

### Aufgabe der Erfindung

Der Erfindung liegt die Aufgabe zugrunde, ein Dichtkissen für eine Atemmaske sowie eine Atemmaske an sich zu schaffen, die sich durch eine hinreichend hohe Dichtwirkung und einen hohen Tragekomfort auszeichnet.

### Erfindungsgemäße Lösung

Diese Aufgabe wird gemäß einem ersten Aspekt der vorliegenden Erfindung gelöst durch eine Maskenkisseneinrichtung für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe, die in Applikationsposition auf der Gesichtsfläche des Maskenanwenders aufsitzt, wobei sich diese Maskenkisseneinrichtung dadurch auszeichnet, dass in dem Maskenkissen querschnittsverdickte Zonen ausgebildet sind, und dass das Maskenkissenmaterial dieser querschnittsverdickten Zonen derart unterschiedliche Materialeigenschaften aufweist, dass die Shore-Härte und/oder die Dichte des Maskenkissens im Randbereich höher ist als im kern- oder zumindest kern-nahen Bereich der querschnittsverdickten Zone.

Dadurch wird es auf vorteilhafte Weise möglich, eine Maskenkisseneinrichtung zu schaffen, die sich durch eine besonders hohe Adaptionsfähigkeit an unterschiedlichste, individuelle Gesichtstekturen auszeichnet.

Gemäß einer besonders bevorzugten Ausführungsform der Erfindung ist die Maskenkisseneinrichtung derart ausgebildet, dass die Shore-Härte des Maskenkissens im Bereich der in Applikationsposition im Stirn- oder Nasenrücken-nahen Umfangszonenbereich niedriger ist, als im Wangen-, Oberlippen- oder Nasenflügeln-nahen Umfangszonenbereich.

Dadurch wird es auf vorteilhafte Weise möglich, insbesondere im Bereich des Nasenrückens die Kisseneinrichtung wirkungsvoll und elastisch gepolstert im Stirn- oder Nasenrückenbereich abzustützen.

Vorzugsweise ist die Maskenkisseneinrichtung derart ausgebildet, dass diese im Bereich der querschnittsverdickten Zonen der Werkstoff gelartige Materialeigenschaften aufweist.

Die unterschiedlichen Werkstoffeigenschaften im Bereich der querschnittsverdickten Zonen können gemäß einer besonders bevorzugten Ausführungsform der Erfindung dadurch erreicht werden, dass die Aushärtung des Maskenkissen-Werkstoffes so erfolgt, dass der Werkstoff zonenweise unterschiedliche Werkstoff-Vernetzungsgrade aufweist.

Die genannten querschnittsverdickten Zonen können in der Maskenkisseneinrichtung derart ausgebildet sein, dass diese unmittelbar an die Dichtlippe angrenzen. Dadurch wird es möglich, die Funktion der Dichtlippe im wesentlichen auf die Herbeiführung einer hinreichenden Dichtwirkung zu beschränken und über die querschnittsverdickten Zonen die Atemmaskenanordnung auf der Gesichtsfläche des Maskenanwenders abzustützen. Alternativ zu der vorangehend beschriebenen Maßnahme - oder bei zonenweise unterschiedlicher Ausgestaltung der Radial-Schnittgeometrie der Maskenkisseneinrichtung - ist es auch möglich, die querschnittsverdickten Zonen so auszubilden, dass diese auf der Gesichtsfläche des Maskenanwenders oder auf einer der Gesichtsfläche des Maskenanwenders abgewandten Innenseite der Dichtlippe aufsitzen.

Die unterschiedlichen Shore-Härten oder Elastizitäts-Module der Maskenkisseneinrichtung, insbesondere im Bereich der querschnittsverdickten Zonen können auch dadurch herbeigeführt werden, dass zur Bildung der Maskenkisseneinrichtung unterschiedlich vorbereitetes Elastomer-Compound-Material verwendet wird. Dieses unterschiedliche Elastomer-Compound-Material kann durch separate Speiseöffnungen in einen entsprechenden Formraum eines Formwerkzeuges eingebracht werden. Die Einspeisung der unterschiedlich vorbereiteten Elastomer-Materialien kann in zeitlich abfolgenden Schritten erfolgen.

Die erfindungsgemäße Maskenkisseneinrichtung kann derart ausgebildet sein, dass diese beispielsweise über einen Umfangsrändabschnitt in abdichtender Weise an einen als Hartschale ausgebildeten Gewölbekörper ansetzbar ist. Dadurch wird es möglich, die Maskenkisseneinrichtung zu Reinigungs- oder Ersatzzwecken von der Hartschale abzunehmen.

Alternativ zu der vorangehend beschriebenen Maßnahme ist es auch möglich, die Maskenkisseneinrichtung integral mit dem Gewölbekörper auszubilden. Hierbei wird die Ausbildung eines Spaltbereiches zwischen der Maskenkisseneinrichtung und dem Gewölbekörper vermieden.

Eine besonders hohe Adaptionsfähigkeit der erfindungsgemäßen Maskenkisseneinrichtung kann dadurch erreicht werden, dass die querschnittsverdickten Zonen zumindest abschnittsweise in Applikationsrichtung federnd aufgehängt sind. Diese federnde Aufhängung der querschnittsverdickten Zonen kann insbesondere über eine Balgstruktur, die beispielsweise als Falten- oder Rollbalg ausgebildet sein kann, erreicht werden. Es ist auch möglich, die Gesichtsdichtlippe über eine Falten- oder Rollbalgstruktur mit den querschnittsverdickten Zonen zu verbinden.

Die Maskenkisseneinrichtung kann so ausgebildet sein, dass die Radial-Querschnitte d.h. die Querschnitte der Maskenkisseneinrichtung variieren.

Die vorangehend beschriebene Maskenkisseneinrichtung bildet Bestandteil einer Atemmaske, die in Applikationsposition den Nasen- und/oder den Mundbereich eines Maskenanwenders übergreift. Sie kann in entsprechender Ausbildung bei einer Nasal-Maske oder auch bei einer Mund- oder Vollgesichtsmaske Anwendung finden.

Die erfindungsgemäße Gestaltung des Querschnitts der Elastomerstrukturen kann auch bei einem Stirnauflageelement Anwendung finden. So ist es gemäß einem weiteren, auch alternativen Lösungsgedanken möglich, Stirnauflage-Pads derart auszubilden, daß deren Verformungsverhalten durch Elastomerzonen verminderter Shore-Härte, oder durch Zonen erhöhten Porenvolumens geprägt ist.

Hinsichtlich eines Verfahren zur Herstellung der erfindungsgemäßen Maskenkisseneinrichtung wird die eingangs angegebene Aufgabe gelöst, durch ein Verfahren bei welchem im Rahmen eines Elastomer-Material-Einbringschrittes das Elastomermaterial in einen Dichtkissenformraum eingebracht wird, wobei die Temperaturverteilung der Formrauminnenwand sowie die Formschliesszeit derart abgestimmt werden, dass das in dem Formraum zu der Dichtkisseneinrichtung vernetzende Elastomermaterial unterschiedliche Shore-Härten erhält.

Es ist möglich, den Vernetzungsvorgang definiert abzubremsen, indem dem Elastomermaterial ein Katalyse-Blocker zugesetzt wird, der ab einem vorgegebenen Vernetzungsgrad, oder Vernetzungszeitraum, eine weitere Vernetzung unterbindet. Der Katalyse-Blocker ist vorzugsweise derart konfiguriert, daß dieser z.B durch UV-Belichtung, oder durch Mikrowellenaufheizung aktiviert wird. Durch die Verwendung eines Katalyse- oder Vernetzungs-Blockers wird es möglich, im Bereich jener Zonen mit niedrigem Vernetzungsgrad, diesen Zustand über einen hinreichend langen Zeitraum - Insbesondere auch unbeschadet einer Nachtemperphase - aufrecht zu erhalten.

Vorzugsweise wird hierbei das Temperaturprofil der Formrauminnenwand derart abgestimmt, dass die in dem Formraum gebildete Dichtkisseneinrichtung in ihrem Verlauf in Umfangsrichtung unterschiedliche Shore-Härten aufweist.

Das Temperaturprofil der Formrauminnenwand wird in vorteilhafter Weise weiterhin derart abgestimmt; dass die Maskenkisseneinrichtung in einem in Applikationsposition dem Stirn- oder Nasenrücken-nahen Bereichen niedrigere Shore-Härten erhält.

Es ist möglich, das teilweise unvernetzte Elastomermaterial abzusaugen oder auszublasen und das Maskenkissen nachzutempern. Auf diese Weise wird es möglich, in der Dichtkisseneinrichtung Kavernen- oder Schlauchzonen auszubilden.

In verfahrenstechnisch weiterhin besonders vorteilhafter Weise wird das Temperaturprofil der Formrauminnenwand während des Elastomermaterial-Einbringschnittes derart variiert, dass dieses zunächst eine erste gegebenenfalls weitgehend konstante Temperaturverteilung aufweist, wobei während der Formschliesszeit die Temperaturverteilung derart verändert wird, dass in ausgewählten Formraumzonen niedrigere Vernetzungsgrade und damit geringere Shore-Härten erreicht werden.

Insbesondere ist es möglich, die Temperaturverteilung des Formwerkzeuges derart abzustimmen, dass das Temperaturprofil während des Elastomer-Material-Einbringschrittes, und über eine sich daran anschließende Haltezeit eine erste Hochtemperaturverteilung hat, wobei nach Ablauf dieser Haltezeit ausgewählte Zonen der Formrauminnenwandung auf niedrigere Temperaturen abgekühlt werden.

In werkzeugtechnischer Hinsicht wird die eingangs angegebene Aufgabe gelöst durch ein Formwerkzeug zur Herstellung einer Maskenkisseneinrichtung, das in Werkzeugschliessstellung einen durch eine Formrauminnenwandung begrenzten, zu der zu bildenden Dichtkisseneinrichtung komplementären Forminnenraum, und eine Heizeinrichtung zur Aufheizung der Formrauminnenwandung aufweist, wobei das Formwerkzeug derart ausgebildet ist, dass sich an der Formrauminnenwandung für unterschiedliche Zonen der darin zu bildenden Maskenkisseneinrichtung ein vorbestimmtes Temperaturprofil ergibt, das zu unterschiedlich hohen Vernetzungsgraden des in dem Forminnenraum aushärtenden Elastomermateriales führt.

Das erfindungsgemäße Formwerkzeug ist vorzugsweise derart ausgebildet, dass das Temperaturprofil derart einstellbar ist, dass die Formraumtemperatur in einem zur Ausbildung des, den Stirn- oder Nasenrückenbereich abdichtenden Abschnitts der Dichtkisseneinrichtung, niedriger ist als die Temperatur in einem zur Ausbildung eines Oberlippen- oder Kinn-Dichtbereiches der Dichtkisseneinrichtung.

Eine im Hinblick auf eine besonders günstige Temperaturverteilung der Formrauminnenwandung vorteilhafte Ausführungsform des Formwerkzeuges, ist dadurch gegeben, dass das Formwerkzeug mit Kühlkanälen versehen ist, zur Abkühlung ausgewählter Abschnitte des Formwerkzeuges beispielsweise über ein zeitgesteuert eingebrachtes Kühlmedium.

Die Erfindung betrifft gemäß einem weiteren Aspekt auch eine Dichtungsstruktur und ein Verfahren zur Herstellung derselben. Insbesondere betrifft die Erfindung Dichtungsstrukturen zur abdichtenden Überbrückung oder Abdichtung, eines Spaltbereiches beispielsweise bei Rohr-Verbindungen, Gehäuseeinrichtungen, sowie Tür- und Fensteranordnungen.

Üblicherweise ist bei derartigen Dichtungsstrukturen ein Dichtflächenabschnitt vorgesehen, der aus einem Elastomer-Material gefertigt ist und in elastisch nachgiebiger Weise an einer Anlagefläche aufsitzt.

Bei derartigen Dichtungsstrukturen besteht das Problem, dass die geforderte Dichtwirkung unter Umständen erst bei vergleichsweise hohen Flächenpressungen erreicht wird.

Der Erfindung liegt die Aufgabe zugrunde, eine Dichtungsstruktur zu schaffen, die unter fertigungstechnischen Gesichtspunkten günstig herstellbar ist, und die sich durch eine hohe Dichtwirkung und hohe Adaptionsfähigkeit auszeichnet.

Diese Aufgabe wird erfindungsgemäß gelöst durch eine Dichtungsstruktur mit einem aus einem Elastomer-Profilmaterial gefertigten Basiskörper, wobei der Basiskörper einen Profilquerschnitt mit wenigstens einer querschnittsverdickten Zone aufweist und das Elastomermaterial derart verarbeitet ist, dass die Shore Härte des Profilmateriales im Randbereich der querschnittsverdickten Zone höher ist, als im Kern oder kernnahen Bereich der querschnittsverdickten Zone.

Dadurch wird es auf vorteilhafte Weise möglich, eine Dichtungsstruktur zu schaffen, die sich auch im Bereich der querschnittsverdickten, Zone durch eine hohe Adaptionsfähigkeit auszeichnet.

Gemäß einer besonders bevorzugten Ausführungsform der Erfindung ist die Dichtungsstruktur derart ausgebildet, dass diese im Bereich der querschnittsverdickten Zonen gel-artige Materialeigenschaften aufweist. Die unterschiedlichen Werkstoffeigenschaften im Bereich der querschnittsverdickten Zone, können gemäß einer besonders bevorzugten Ausführungsform der Erfindung dadurch erreicht werden, dass die Aushärtung des Profilmateriales derart erfolgt, dass das Profilmaterial zonenweise unterschiedliche Werkstoff-Vernetzungsgrade aufweist.

Der Profilquerschnitt der Dichtungsstruktur kann derart gestaltet sein, dass die querschnittsverdickte Zone unmittelbar an eine integrale Dichtlippe angrenzt.

Die unterschiedlichen Shore-Härten oder Elastizitätsmodule des Profilmateriales, insbesondere im Bereich der querschnittsverdickten Zone, können dadurch herbeigeführt werden, dass die Dichtungsstruktur aus unterschiedlich vorbereiteten Elastomer-Mischungen (Compound-Material) gefertigt wird. Diese unterschiedlichen Elastomer-Compound Systeme können durch seperate Speiseöffnungen in einen Formraum eines Formwerkzeuges, insbesondere in einen Extrusionskanal eingebracht werden. Die Einspeisung der unterschiedlich vorbereiteten Elastomer-Compoundmischungen kann in zeitlich abfolgenden Schritten erfolgen.

Die erfindungsgemäße Dichtungsstruktur kann derart ausgebildet sein, dass diese beispielsweise als umlaufender Dichtring bei einer Rohr-Verbindung vorgesehen ist.

Hinsichtlich eines Verfahrens zur Herstellung der erfindungsgemäßen Dichtungsstruktur, wird die eingangs angegebene Aufgabe gelöst, durch ein Verfahren, bei welchem im Rahmen eines Elastomer-Materialeinbringschrittes das Elastomer-Material in einen Dichtungsstruktur-Formraum eingebracht wird, wobei die Aufheizung des Elastomer-Materiales in dem Formraum derart abgestimmt erfolgt, dass das Profilmaterial im Bereich einer querschnittsverdickten Zone einen niedrigeren Vernetzungsgrad erhält, als im Randbereich der querschnittsverdickten Zone oder verbleibenden dünnwandigen Abschnitten der Dichtungsstruktur.

Es ist möglich, den Vernetzungsvorgang definiert abzubremsen, indem dem Profilmaterial ein Katalyse-Blocker zugesetzt wird, der ab einem vorgegebene Vernetzungsgrad oder Vernetzungszeitraum eine weitere Vernetzung unterbindet. Dieser Katalyse-Blocker ist vorzugsweise derart konfiguriert, dass dieser zum Beispiel durch UV-Belichtung oder durch Mikrowellenaufheizung aktiviert wird. Durch die Verwendung eines Katalyse- oder Vernetzungs-Blocker wird es möglich, im Bereich jener Zonen mit niedrigem Vernetzungsgrad diesen Zustand über einen hinreichend langen Zeitraum, insbesondere auch unbeschadet einer Nachtemperphase aufrechtzuerhalten.

Vorzugsweise wird das Temperaturprofil einer zur Bildung der Dichtungsstruktur vorgesehenen Formrauminnenwand derart abgestimmt, dass die in dem Formraum gebildete Dichtungsstruktur Abschnitte unterschiedlicher Shore-Härte aufweist.

Es ist möglich, das im Bereich der querschnittsverdickten Zone gegebenenfalls unvernetzte Elastomer-Material zumindest teilweise abzusaugen oder auszublasen und die Dichtungsstruktur anschließend nachzutempern. Auf diese Weise wird es möglich, in der Dichtungsstruktur Kavernen- oder Schlauchzonen auszubilden.

Weitere Einzelheiten und Merkmale der angegebenen Erfindungen ergeben sich aus der nachfolgenden Beschreibung in Verbindung mit der Zeichnung.

Die nachfolgenden Punkte sind bevorzugte Ausführungsformen der vorliegenden Erfindung:
1. Maskenkisseneinrichtung für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe, die in Applikationsposition auf der Gesichtsfläche des Maskenanwenders aufsitzt, dadurch gekennzeichnet, dass in dem Maskenkissen wenigstens eine querschnittsverdickte Zone ausgebildet ist, und dass das Maskenkissenmaterial dieser querschnittsverdickten Zone derart unterschiedliche Materialeigenschaften aufweist, dass die Shore-Härte des Maskenkissens im Randbereich der querschnittsverdickten Zone höher ist als in deren kern- oder zumindest kernnahen Bereich.
2. Maskenkisseneinrichtung nach Punkt 1, dadurch gekennzeichnet, dass die Shore-Härte des Maskenkissens im Bereich der in Applikationsposition im Stirn- oder Nasenrücken-nahen Zonen niedriger als im Bereich der in Applikationsposition den Wangen-, Oberlippen- oder Nasenflügeln-nahen Umfangszonen.
3. Maskenkisseneinrichtung nach Punkt 2, dadurch gekennzeichnet, dass im Kernbereich der querschnittsverdickten Zonen der Werkstoff gelartige Materialeigenschaften aufweist.
4. Maskenkisseneinrichtung nach wenigstens einem der Punkte 1 bis 3, dadurch gekennzeichnet, dass die unterschiedlichen Werkstoffeigenschaft im Bereich der querschnittsverdickten Zonen durch unterschiedliche Werstoff-Vernetzungsgrade verursacht sind.
5. Maskenkisseneinrichtung nach wenigstens einem der Punkte 1 bis 4, dadurch gekennzeichnet, dass die querschnittsverdickten Zonen an die Dichtlippe angrenzen.
6. Maskenkisseneinrichtung nach wenigstens einem der Punkte 1 bis 5, dadurch gekennzeichnet, dass die querschnittsverdickten Zone in Applikationsposition zumindest abschnittsweise auf der Gesichtsfläche des Maskenanwenders aufsitzt.
7. Maskenkisseneinrichtung nach wenigstens einem der Punkte 1 bis 6, dadurch gekennzeichnet, dass die querschnittsverdickte Zone in Applikationsposition zumindest abschnittsweise auf der, der Gesichtsfläche des Maskenanwenders abgewandten Innenseite der Gesichts-Dichtlippe aufsitzt.
8. Maskenkisseneinrichtung nach wenigstens einem der Punkts 1 bis 7, dadurch gekennzeichnet, dass die querschnittsverdickten Zonen aus wenigstens zwei unterschiedlich vorbereiteten Elastomer-Copound-Materialien gebildet sind.
9. Maskenkisseneinrichtung nach wenigstens einem der Punkte 1 bis 8, dadurch gekennzeichnet, dass diese an einen durch eine Hartschale gebildete Gewölbekörper ansetzbar ausgebildet ist.
10. Maskenkisseneinrichtung nach wenigstens einem der Punkte che 1 bis 9, dadurch gekennzeichnet, dass die Dichtkisseneinrichtung integral mit dem Gewölbekörper ausgebildet ist.
11. Maskenkisseneinrichtung nach wenigstens einem der Punkte che 1 bis 10, dadurch gekennzeichnet, dass die querschnittsverdickten Zonen zumindest abschnittsweise in Applikationsrichtung federnd nachgiebig aufgehängt ist.
12. Maskenkisseneinrichtung nach wenigstens einem der Punkte che 1 bis 11, dadurch gekennzeichnet, dass die Dichtlippe über eine Faltenstruktur federnd mit der querschnittsverdickte Zone gekoppelt ist.
13. Atemmaske mit einem in Applikationsposition den Nasen- und/oder den Mundbereich eines Maskenanwenders übergreifenden Gewölbekörper und einer Maskenkisseneinrichtung nach wenigstens einem der Punkte 1 bis 12.
14. Atemmaske nach Punkt 13, dadurch gekennzeichnet, dass der Gewölbekörper integral mit der Dichtkisseneinrichtung ausgebildet ist.
15. Atemmaske nach Punkt 13, dadurch gekennzeichnet, dass der Gewölbekörper durch eine Hartschale gebildet ist.
16. Verfahren zur Herstellung einer Maskenkisseneinrichtung einer Atemmaske, bei welchen im Rahmen eines Elastomer-Material-Einbringschrittes, das Elastomermaterial in einen Maskenkissenformraum eingebracht wird, wobei die Temperaturverteilung der Formrauminnenwand sowie die Formschliesszeit derart abgestimmt werden, dass das in dem Formraum zu der Maskenkisseneinrichtung vernetzende Elastomermaterial, unterschiedliche Shore-Härten erhält.
17. Verfahren nach Punkt 16, dadurch gekennzeichnet, dass das Temperaturprofil der Formrauminnenwand derart abgestimmt wird, dass die in dem Formraum gebildete Dichtkisseneinrichtung in ihrem Verlauf in Umfangsrichtung unterschiedliche Shore-Härten aufweist.
18. Verfahren nach Punkt 16 bis 17, dadurch gekennzeichnet, dass das Temperaturprofil derart abgestimmt wird, dass die Maskenkisseneinrichtung in einem in Applikationsposition dem Stirn- oder Nasenrücken-nahen Bereich niedrigere Shore-Härten erhält.
19. Verfahren nach Punkt 16 bis 18, dadurch gekennzeichnet, dass das Temperaturprofil der Formrauminnenwand während des Elastomer-Material-Einbringschrittes eine erste Temperatur-Profilcharakteristik aufweist und dass die Temperaturcharakteristik während der Formschliesszeit verändert wird.
20. Verfahren nach Punkt 19, dadurch gekennzeichnet, dass das Temperaturprofil während des Elastomer-Material-Einbringschrittes eine erste insbesondere konstante, Hochtemperaturverteilung hat, und dass während der Formschliesszeit ausgewählte Zonen der Formrauminnenwandung auf niedrigere Temperaturen abgekühlt werden.
21. Formwerkzeug zur Herstellung einer Maskenkisseneinrichtung mit einem in Werkzeugschliessstellung, einen durch einen durch eine Formrauminnenwandung begrenzten, zu der zu bildenden Maskenkisseneinrichtung komplementären Forminnenraum und einer Heizeinrichtung zur Aufheizung der Formrauminnenwandung, dadurch gekennzeichnet, dass das Formwerkzeug derart ausgebildet ist, dass sich an der Formrauminnenwandung ein vorbestimmtes Temperaturprofil ergibt.
22. Formwerkzeug nach Punkt 21, dadurch gekennzeichnet, dass das Temperaturprofil derart einstellbar ist, dass die Formraumtemperatur in einem Formraumabschnitt zur Ausbildung eines den Stirn- oder Nasenrückenbereich abdichtenden Abschnitts der Maskenkisseneinrichtung niedriger ist als die Temperatur in einem zur Ausbildung eines Oberlippen- oder Kinn-Dichtbereiches der Maskenkisseneinrichtung vorgesehenen Formraumabschnitt.
23. Formwerkzeug nach einem der Punkte 19 oder 20, dadurch gekennzeichnet, dass dieses Kühleinrichtungen umfasst, zur Abkühlung ausgewählter Abschnitte des Formwerkzeuges.
24. Formwerkzeug nach wenigstens einem der Punkte 19 bis 21, dadurch gekennzeichnet, dass das. Temperaturprofil während der Formschließzeit veränderbar ist.
25. Maskenkisseneinrichtung für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe, die in Applikationsposition auf der Gesichtsfläche des Maskenanwenders aufsitzt, dadurch gekennzeichnet, dass in dem Maskenkissen wenigstens eine querschnittsverdickte Zone ausgebildet ist, und dass das Maskenkissenmaterial in dieser querschnittsverdickten Zone derart geschäumt ausgebildet ist, dass das Maskenkissens im Bereich der querschnittsverdickten ein Schaumploster bildet.
26. Maskenkisseneinrichtung für eine Atemmaske mit einer Aufnahmeöffnung, die in Applikationsposition der Atemmaske wenigstens mit dem Nasen- und/oder Mundöffnungsbereich eines Maskenanwenders übereinkommt, und einer aus einem elastomeren Material gebildeten, um die Aufnahmeöffnung umlaufenden Dichtlippe, die in Applikationsposition auf der Gesichtsfläche des Maskenanwenders aufsitzt, dadurch gekennzeichnet, dass im Innenbereich Maskenkissen wenigstens eine querschnittsverdickte Zone ausgebildet ist, und dass das Maskenkissenmaterial dieser querschnittsverdickten Zone gelartige Materialeigenschaften aufweist, wobei die querschnittsverdickte Zone durch Einsetzen eines gelartig vernetzten Elastomerkörper in eine, im Innenraum der Maskenkisseneinrichtung vorgesehene Fixierstruktur gebildet ist.
27. Maskenkisseneinrichtung nach Punkt 26, dadurch gekennzeichnet, daß der gelartig vernetzte Körper eine hufeisenartige Gestalt aufweist und sich in Applikationsposition über den Nasenrücken des Maskenanwenders erstreckt.
28. Maskenkisseneinrichtung nach Punkt 26, dadurch gekennzeichnet, daß der gelartig vernetzte Körper ringartig der Dichtlippe folgend, um eine Mund- und/oder Nasenöffnung umlaufend ausgebildet ist.
29. Maskenkisseneinrichtung für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe, die in Applikationsposition auf der Gesichtsfläche des Maskenanwenders aufsitzt, dadurch gekennzeichnet, dass in dem Maskenkissen wenigstens ein Aufnahmetaschenabschnitt ausgebildet ist und in diesen Aufnahmetaschenabschnitt ein Polsterkörper aus einem Gel- oder Schaummaterial vorgesehen ist, wobei der Verlauf des Taschenabschnitts derart festgelegt ist, dass die Dichtlippeneinrichtung eine Hautkontaktzone (GS1) definiert in welcher die Dichtlippeneinrichtung durch den Polsterkörper gegen die Gesichtsfläche gedrängt wird.
30. Maskenkisseneinrichtung nach Punkt 29, dadurch gekennzeichnet, dass der Polsterkörper in Segmente unterteilt ist.
31. Stirnpad für eine Atemmaske mit einem Stirnpadbasiskörper der aus einem elastomeren Material gefertigt ist und einen Koppelungsabschnitt aufweist, zur Festlegung des Stirnpads an einer Stirnauflageeinrichtung und einen Schaftabschnitt zur radial nachgiebigen Lagerung eines zur Auflage auf einer Stirnfläche vorgesehenen Stirnkontaktabschnitts aufweist, wobei das Stirnpad eine querschnittsverdickte Zone aufweist und in dieser Zone mit einem Gel- oder Schaummaterial versehen ist.
32. Dichtungsstruktur mit einem aus einem elastomeren Profilmaterial gefertigten Basiskörper, wobei der Basiskörper einen Profilquerschnitt mit wenigstens einer querschnittsverdickten Zone aufweist und das Elastomermaterial derart verarbeitet ist, dass die Shore-Härte des Profilmateriales im Randbereich der querschnittsverdickten Zone höher ist als im Kern - oder kernnahen Bereich der querschnittsverdickten Zone.
33. Dichtungsstruktur nach Punkt 32, dadurch gekennzeichnet, dass Kernbereich der querschnittsverdickten Zone der Werkstoff gel-artige Materialeigenschaften aufweist.
34. Dichtungsstruktur nach Punkt 32 oder 33, dadurch gekennzeichnet, dass die unterschiedlichen Werkstoffeigenschaften im Bereich der querschnittsverdickten Zone durch unterschiedliche Werkstoffvernetzungsgrade verursacht sind.
35. Dichtungsstruktur nach wenigstens einem der Punkte 32 bis 34, dadurch gekennzeichnet, dass diese eine Dichtlippe umfasst, und dass die querschnittsverdickte Zone an die Dichtlippe angrenzt.
36. Dichtungsstruktur nach wenigstens einem der Punkte 32 bis 35, dadurch gekennzeichnet, dass die querschnittsverdickte Zone aus wenigstens zwei unterschiedlich vorbereiteten Elastomer-Compoundsystemen gebildet ist.
37. Dichtungsstruktur nach wenigstens einem der Punkte 32 bis 36, dadurch gekennzeichnet, dass die Dichtungsstruktur eine Befestigungsprofilierung aufweist.
38. Dichtungsstruktur nach wenigstens einem der Punkte 32 bis 37, dadurch gekennzeichnet, dass die Dichtungsstruktur einen Faltenbalgabschnitt aufweist, der integral mit der querschnittsverdickten Zone ausgebildet ist.
39. Verfahren zur Herstellung einer Dichtungsstruktur, bei welchem im Rahmen eines Elastomer-Material-Einbringschrittes das Elastomermaterial in einen Dichtungsstruktur-Formraum oder Dichtungsextrusionskanal eingebracht wird, wobei die Temperaturverteilung in dem Dichtungsstruktur-Formraum oder in dem Extrusionskanal sowie die Verweilzeit derart abgestimmt werden, dass das zu der Dichtungsstruktur vernetzende Elastomermaterial im Bereich einer querschnittsverdickten Zone unterschiedliche Shore-Härten erhält.
40. Verfahren nach Punkt 39, dadurch gekennzeichnet, dass im Kernbereich der querschnittsverdickten Zone die Vernetzung des Profil-Materiales gebremst oder blockiert wird.
41. Verfahren nach Punkt 40, dadurch gekennzeichnet, dass die Abbremsung des Vernetzungsvorganges durch UV-Belichtung verzögert wird.
42. Verfahren nach Punkt 41, dadurch gekennzeichnet, dass die Abbremsung des Vernetzungsvorganges durch Mikrowellenbestrahlung verzögert wird.
43. Verfahren nach Punkt 42, dadurch gekennzeichnet, dass die Abbremsung des Vernetzungsvorganges durch elektromagnetische Strahlung verzögert wird.
44. Verfahren nach Punkt 43, dadurch gekennzeichnet, dass die Vernetzung im Bereich der querschnittsverdickten Zone durch Injektion eines Katalyse-Blockers in den Kernbereich gebremst wird.

### Kurzbeschreibung der Figuren

Es zeigt:
- **Fig. 1**: einen Querschnitt durch eine Dichtkisseneinrichtung gemäß einer ersten Ausführungsform der Erfindung mit ei- ner querschnittsverdickten Zone und darin enthaltenen Bereichen unterschiedlicher Shore-Härte;
- **Fig.**: **2** eine Schnittansicht durch eine Dichtkisseneinrichtung ebenfalls mit einer querschnittsverdickten Zone und dar- in ausgebildeten Bereichen unterschiedlicher Materialei- genschaften;
- **Fig. 3**: ein Diagramm zur Erläuterung der unterschiedlichen Temperaturverteilung einer Formrauminnenwand eines Form- werkzeuges zur Herstellung einer Dichtkisseneinrichtung mit in Umfangsrichtung variierender Shore-Härte.
- **Fig. 4**: eine Schnittansicht zur Erläuterung des Querschnitts einer Dichtkisseneinrichtung mit einem, in deren Innen- bereich, einer Dichtlippe benachbart angeordneten, ge- lartig vernetzten Einsatzelement.
- **Fig. 5a**: eine Variante des Einsatzelementes 16 nach welcher deren gelartig vernetzter Körper eine hufeisenartige Gestalt aufweist und sich in Applikationsposition über den Na- senrücken des Maskenanwenders erstreckt.
- **Fig. 5b**: zeigt eine Variante des Einsatzelementes 16 nach welcher deren gelartig vernetzte Körper ringartig der Dichtlippe folgend, um eine Mund- und/oder Nasenöffnung umlaufend ausgebildet ist;
- **Fig. 6a**: eine Schnittansicht durch eine Dichtkisseneinrichtung ebenfalls mit einer querschnittsverdickten und einem Gel- oder Schaummaterial ausgefüllten Zone die hier durch einen integral mit der Dichtlippeneinrichtung ge- bildeten Taschenabschnitt gebildet ist, wobei der Ta- schenabschnitt durch ein Rahmenelement abgedeckt ist und die Dichtlippeneinrichtung mit einem Schalenkörper ver- sehen ist, der integral mit der Dichtlippeneinrichtng aus einem Elastomermaterial gefertigt ist;
- **Fig. 6b** eine: Schnittansicht durch eine Dichtkisseneinrichtung ähnlich Figur 6b, wobei das Rahmenelement eine An- schlussstruktur bildet, zur Ankoppelung einer den Nasen- bereich überdeckenden Maskenschale;
- **Fig. 6c**: eine weitere Schnittansicht durch eine Dichtkissen- einrichtung ebenfalls mit einer querschnittsverdickten und mit einem nachgiebigen Gel- oder Schaummaterial ge- füllten querschnittsverdickten Zone;
- **Fig. 7**: eine vereinfachte perspektivische Darstellung eines aus einem Gel- oder Schaummaterial bestehenden Polsterkör- pers zur Abstützung einer Atmmaske;
- **Fig.**: **8** eine vereinfachte Seitenansicht zur Erläuterung eines möglichen Verlaufs eines Polsterkörpers nach Fig. 7 auf der Gesichtsfläche eines Maskenanwenders;
- **Fig. 9**: eine vereinfachte perspektivische Darstellung eines Gel- oder Schaumpolsterkörpers, der in verschiedene Segmente unterteilt ist, wobei die Segmente unterschiedliche me- chanische Eigenschaften, insbesondere unterschiedliche Shorehärten aufweisen und die Grenzflächen organisch un- eben ausgebildet sind;
- **Fig. 10**: eine räumliche Darstellung zur Erläuterung einer Polsterkörpervariante mit einem im Nasenrückenüberbrü- ckungsbereich ausgesparten Abschnitt;
- **Fig. 11**: eine Schnittansicht eines den Nasenrückenbereich überbrückenden Polsterkörpersegmentes;
- **Fig. 12**: eine Skizze zur Illustration des Verlaufs eines Polsterkörpers auf der Gesichtsfläche eines Patienten;
- **Fig. 13**: eine Skizze zur Erläuterung eines auf dem Oberlippen- bereich eines Patienten aufsitzenden Polsterkörperseg- ments;
- **Fig. 14**: eine perspektivische Ansicht einer Dichtlippenein- richtung einschließlich zugehörigem Maskenbasisköpers,

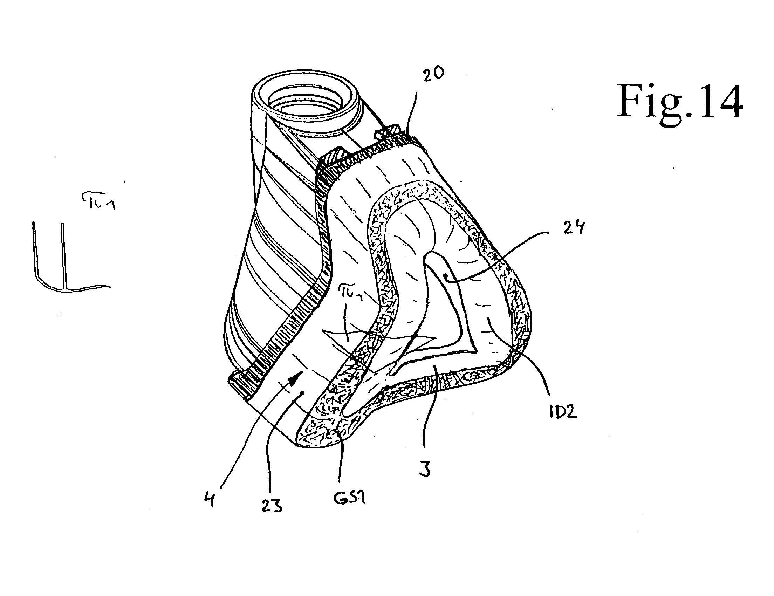

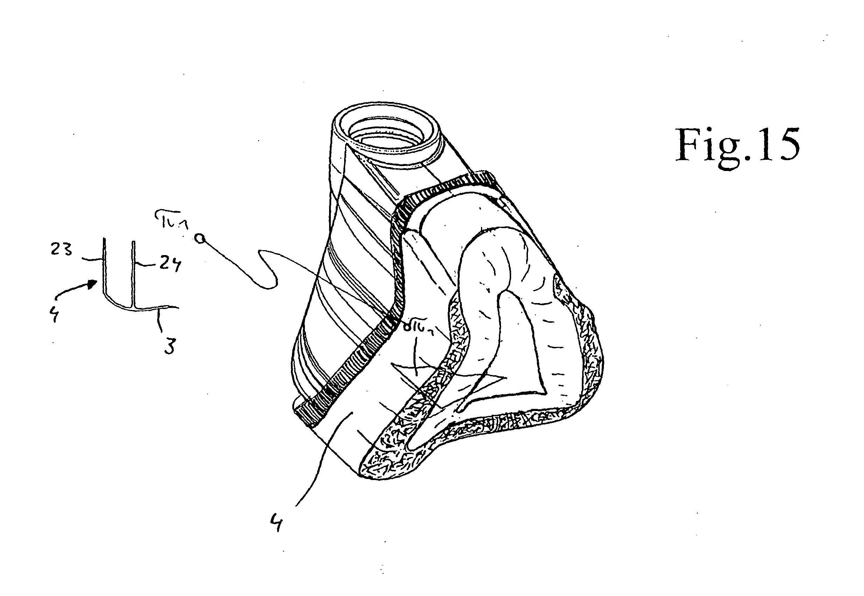

- **Fig. 15**: wobei die Dichtlippeneinrichtung einen Umfangstaschenab- schnitt aufweist der mit einem Gel- oder Schaumstoffma- terial gefüllt ist und im Bereich der abgesetzt gekenn- zeichneten Dichtlippenumfangszone den Maskenbasiskörper auf der Gesichtsfläche des Patienten abstützt; eine perspektivische Ansicht einer Dichtlippenein- richtung ähnlich Figur 14 einschließlich zugehörigem Maskenbasisköpers, wobei die Dichtlippeneinrichtung ei- nen Umfangstaschenabschnitt aufweist der mit einem Gel- oder Schaumstoffmaterial gefüllt ist und im Bereich der abgesetzt gekennzeichneten Dichtlippenumfangszone den Maskenbasiskörper auf der Gesichtsfläche des Patienten abstützt und im Berich des Nasenrückens mit einer Aus- nehmung versehen ist, oder unterbrochen ist, so dass in dieser Zone keine Abstützung über das Gel- oder Schaum- stoffmaterial auf dem Nasenrücken erfolgt;
- **Fig. 16**: eine perspektivische Ansicht der Dichtlippeneinrich- tung nach Figur 15 zur Illustration des partiell unter- brochenen Umfangstaschenabschnitts;
- **Fig. 17**: eine perspektivische Ansicht einer Atemmaske mit schwenkbewegbar angelenkter Stirnauflageeinrichtung und einer Dichtlippeneinrichtung einschließlich zugehörigem Maskenbasisköpers, gemäß Figuren 14 oder 15;
- **Fig. 18a**: **bis 18e** Schnittansichten von Varianten von Stri- nauflage-Pads insbesondere zur Verwendung in Verbindung mit einer Atemmaske, beispielsweise gemäß Figur 17, wo- bei die Stirnauflagepads Querschnittsverdickte Zonen aufweisen die mit einem Gel- oder Schaumstoffmaterial gefüllt sind;
- **Fig. 19**: eine perspektivische Ansicht eines Abschnitts einer erfindungsgemäßen Dichtungsstruktur mit einer quer- schnittsverdickten Zone und darin ausgebildeten Berei- chen niedriger Shore-Härte.

### Ausführliche Beschreibung der Figuren

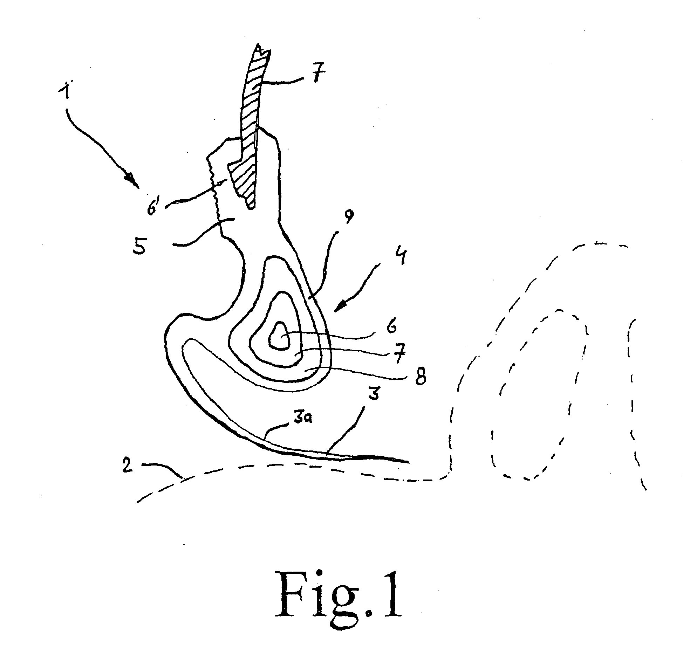

Die in Fig. 1 dargestellte Dichtkisseneinrichtung 1 umfasst eine in Applikationsposition auf einer hier angedeuteten Gesichtsfläche 2 aufsitzenden Dichtlippe 3.

Die Maskenkisseneinrichtung 1 umfasst weiterhin eine querschnittsverdickte Zone 4, die bei diesem Ausführungsbeispiel zwischen einem oberen Umfangsrand 5 und der Dichtlippe 3 angeordnet ist.

Die querschnittsverdickte Zone 4 ist derart ausgebildet, dass die Shore-Härte des die querschnittsverdickte Zone bildenden Materiales, variiert. Die hier angedeuteten Materialzonen haben einen nahezu gelartigen Charakter. Die Shore-Härte der am weitesten innen liegenden Materialzone 6 ist niedriger, als, die der angrenzenden Materialzone 7, welche wiederum eine niedrigere Shore-Härte hat, als die angrenzende Materialzone 8. Der Außenbereich 9 der querschnittsverdickten Zone ist aus einem im wesentlichen vollständig vernetzten Elastomermaterial gefertigt und hat im wesentlichen dieselbe Shore-Härte wie die Dichtlippe 3.

Die unterschiedlichen Materialeigenschaften im Bereich der querschnittsverdickten Zone 4 werden durch das Temperaturprofil eines zur Fertigung der Maskenkisseneinrichtung 1 vorgesehenen Formwerkzeuges sowie durch Begrenzung der Verweilzeit, der zumindest im Formraum-nahen Bereich, hinreichend ausgehärteten Maskeneinrichtung in dem Formwerkzeug bestimmt.

Es ist möglich, den Vernetzungsvorgang definiert abzubremsen, indem dem Elastomermaterial ein Katalyse-Blocker zugesetzt wird, der ab einem vorgegebenen Vernetzungsgrad, oder Vernetzungszeitraum, eine weitere Vernetzung unterbindet. Der Katalyse-Blocker ist vorzugsweise derart konfiguriert, daß dieser z.B. durch UV-Belichtung, oder durch Mikrowellenaufheizung aktiviert wird. Durch die Verwendung eines Katalyse- oder Vernetzungs-Blockers wird es möglich, im Bereich jener Zonen mit niedrigem Vernetzungsgrad, diesen Zustand über einen hinreichend langen Zeitraum - Insbesondere auch unbeschadet einer Nachtemperphase - aufrecht zu erhalten.

Bei der hier dargestellten Maskenkisseneinrichtung 1 ist die Dichtlippe 3 derart mit der querschnittsverdickten Zone 4 gekoppelt, dass die querschnittsverdickte Zone 4 in Applikationsposition der Maskenkisseneinrichtung 1 gegebenenfalls auf einer Innenseite 3a der Dichtlippe 3 aufsitzen kann. Hierdurch kann über die querschnittsverdickte Zone 4 die Maskenkisseneinrichtung 1 auf der Gesichtsfläche 2 abgestützt werden, wobei sich auf Grund der besonderen Eigenschaften der querschnittsverdickten Zone 4, nur vergleichsweise geringe Flächenpressungen ergeben.

Vermittels der querschnittsverdickten Zone 4 ist es bei dieser Ausführungsform auch möglich, die Dichtlippe 3 insbesondere in den kritischen Bereichen wie zum Beispiel dem Nasenrückenbereich zusätzlich gegen die Gesichtsfläche des Maskenanwenders zu drängen.

Im Bereich des oberen Umfangsrandes 5 ist hier eine Koppelungsstruktur 6 ausgebildet, über welche die Maskenkisseneinrichtung 1 mit einer hier nur andeutungsweise dargestellten, eine Gewölbekörper bildenden Hartschale 7 in hinreichend abdichtender Weise gekoppelt werden kann.

Die hier dargestellte Maskenkisseneinrichtung 1 ist aus einem Zwei-Komponenten-Silikonmaterial gefertigt, wobei die Materialzone 6 im wesentlichen nicht ausgehärtet ist. Die Materialzonen 7 und 8 sind gelartig-teilausgehärtet, wobei der Vernetzungsgrad der Materialzone 8 größer ist als der Vernetzungsgrad in der Materialzone 7.

Die höheren Vernetzungsgrade im Randbereich der Maskenkisseneinrichtung 1 werden insbesondere durch die hohe Aufheizung des an eine Formraumwandung angrenzenden Silikonmateriales erreicht.

Unabhängig von der vorangehend beschriebenen Ausgestaltung der querschnittsverdickten Zone 4 - oder in besonders vorteilhafter Weise in Kombination mit dieser - ist es möglich, die Dichtlippe 3 derart auszubilden, dass diese in ihrem Umfangsverlauf unterschiedliche Shore-Härten aufweist. Diese unterschiedlichen Shore-Härten können ebenfalls durch willkürliche Festlegung des Temperaturprofiles eines zur Bildung der Maskenkisseneinrichtung 1 vorgesehenen Formwerkzeuges im Bereich seiner Formrauminnenwandung erreicht werden.

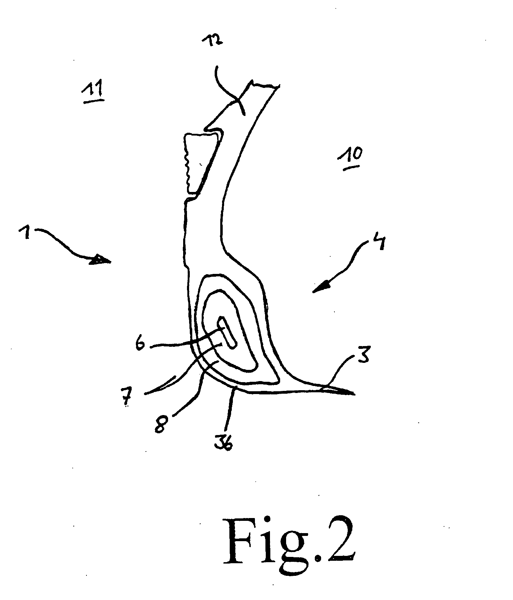

In Fig. 2 ist ein weiteres Ausführungsbeispiel einer Maskenkisseneinrichtung 1 dargestellt, die hier integral mit einem den Maskeninnenraum 10 vom Umgebungsbereich 11 trennenden Gewölbekörper 12 ausgebildet ist.

Die auch bei dieser zweiten Ausführungsform vorgesehene querschnittsverdickte Zone 4 weist ebenfalls Materialzonen 6, 7, 8 auf, die sich hinsichtlich der darin vorherrschenden Shore-Härte unterscheiden. Die querschnittsverdickte Zone sitzt bei diesem Ausführungsbeispiel über eine vollständig vernetzte, und in die Dichtlippe 3 übergehende Aussenwandung 3b auf der hier näher dargestellten Gesichtsfläche des Maskenanwenders ab.

Die Dichtlippe 3 ist hier nur als kleiner, zur Nasen- und/oder Mundöffnung radial einwärts vordringender Dichtlippenabschnitt ausgebildet.

Obgleich hier nicht näher dargestellt, ist es möglich, die querschnittsverdickte Zone 4 und/oder die Dichtlippe 3 über Faltenstrukturen mit dem Gewölbekörper 12 zu koppeln, so dass hierdurch eine nochmals verbesserte Adaptionsfähigkeit der Maskenkisseneinrichtung 1 an die individuelle Gesichtstektur des Maskenanwenders ergibt.

Die querschnittsverdickte Zone 4 kann mit gegebenenfalls variierender Querschnittsgeometrie vollständig umlaufend ausgebildet sein. Alternativ hierzu ist es jedoch auch möglich, die querschnittsverdickte Zone in der Maskenkisseneinrichtung 1 nur im Bereich des, in Applikationsposition dem Stirn-oder Nasenrücken-nahen Bereich auszubilden.

Es ist auch möglich, in Umfangsrichtung der Maskenkisseneinrichtung 1 derart unterschiedliche Radial-Schnittgeometrien vorzusehen, dass beispielsweise im Oberlippen- oder Kinn-nahen Bereich der Querschnitt durch die Maskenkisseneinrichtung 1 im wesentlichen dem in Fig. 2 skizzierten Aufbau entspricht, wogegen im Stirn- oder Nasenrücken-nahen Bereich der Maskenkisseneinrichtung 1 der Querschnitt der Maskenkisseneinrichtung 1 im wesentlichen dem in Fig. 1 dargestellten Querschnitt entspricht.

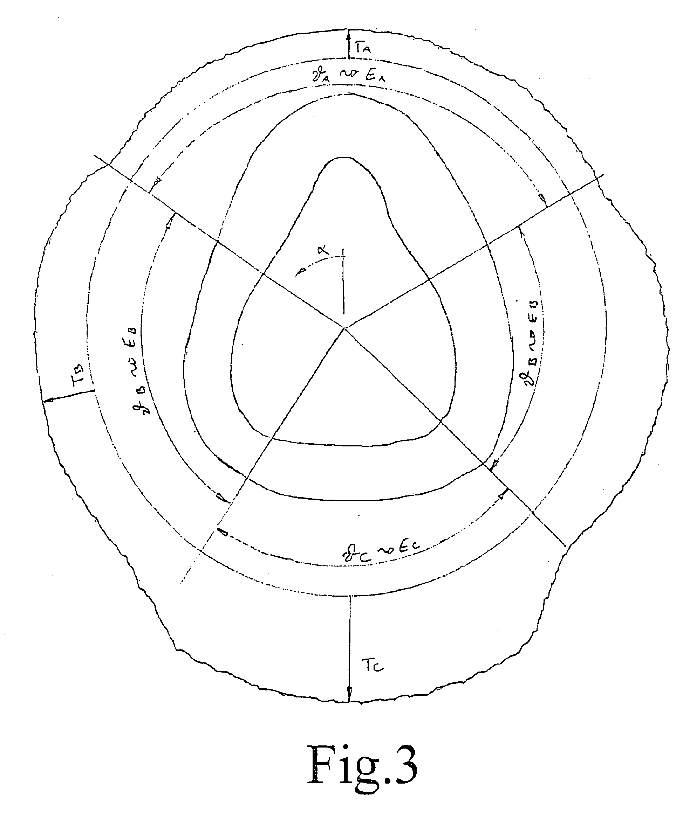

In Fig. 3 ist in der Art eines Polardiagrammes der Temperaturverlauf einer, der Dichtlippeneinrichtung 3 sowie insbesondere der querschnittsverdickten Zone 4 benachbarten Formrauminnenwandung dargestellt. Wie aus diesem Polardiagramm ersichtlich, herrscht in einem hier als □ A gekennzeichneten Polarbereich eine vergleichsweise niedrige Durchschnittstemperatur TA, die zu einem niedrigeren Vernetzungsgrad des Elastomerwerkstoffes der Maskenkisseneinrichtung 1 führt, als die Temperaturen TB und TC in den Bereichen □ B und □ C.

Die Temperatur TC im Bereich ϑC übersteigt die Temperatur TB im Bereich ϑB.

Die Temperatur im Bereich ϑA bestimmt im wesentlichen den Vernetzungsgrad und damit die Shore-Härte der Dichtlippe 3 in einem Stirn- oder Nasenrücken-nahen Bereich.

Die Temperatur TB in den Bereichen ϑB bestimmt im wesentlichen die Shore-Härte in den Wangen oder Nasenflügel-nahen Bereich der Dichtlippe 3. Die Temperatur TC bestimmt im wesentlichen die Shore-Härte der Dichtlippe 3 in ihrem auf dem Kinn- oder Oberlippenbereich des Maskenanwenders aufsitzenden Umfangsabschnitt.

Die hier dargestellte Temperaturverteilung kann während der Verweilzeit des Elastomermateriales in dem entsprechenden Formraum eines Formwerkzeuges verändert werden. Die Änderung des Temperaturprofiles in dem Formraum des Formwerkzeuges kann durch Änderung der Heizleistung oder durch abschnittsweise Kühlung des Formwerkzeuges erfolgen.

Kühl- und/oder Heizorgane können sowohl im Bereich der Aussenwerkzeuge als auch im Bereich der in Schließstellung des Formwerkzeuges in den Außenwerkzeugen unter Belassung des Dichtkissen-Formraumes aufgenommenen Formkernwerkzeugen ausgebildet sein.

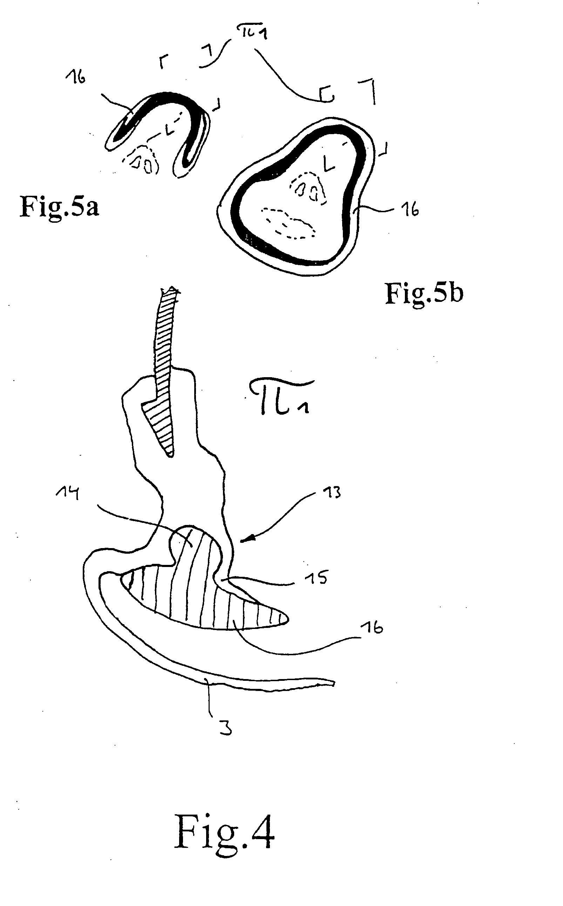

Die in Figur 4 dargestellte Dichtkisseneinrichtung umfaßt ein Einsatzelement 16 das aus einem gelartig ausgehärteten, oberflächlich verhauteten Elastomermaterial gefertigt ist. Dieses Einsatzelement ist über eine Fixierstruktur 13 im Innenbereich der Dichtkisseneinrichtung angeordnet. Die Fixierstruktur 13 ist durch eine Profilierung der Dichtkisseneinrichtung integral mit dieser ausgebildet. Die Profilierung ist derart gestaltet, daß ein Wulstabschnitt 14 des Einsatzelementes 16 durch einen Haltelippenabschnitt 15 gehalten ist. Ein der Dichtlippe 3 zugewandter Abschnitt des Einsatzelementes 16 ist derart ausgebildet, daß dieser ggf. auf einer Innenseite der Dichtlippe 3 aufsitzen kann.

Es ist möglich, die Fixerstruktur im Innenbereich der Dichtkisseneinrichtung derart auszubilden, daß an diese unterschiedlich ausgebildete Einsatzelemente ankoppelbar sind. Es ist möglich, mehrere, Dichtkissen-kompatible Einsatzelemente in Gel- Schaum- und/oder Schlauchbauform vorzusehen und diese anwendungsfallbezogen auszuwählen und eine Atemmaske damit zu bestücken. Es ist möglich, wenigstens eine Variante eines zumindest teilweise vorgeformten, vorzugsweise gelartigen, Einsatzelementes vorzuhalten, das beispielsweise durch Erwärmung in einen hinreichend plastifizierten Zustand bringbar ist, in welchem das Einsatzelement, an die individuelle Gesichtstektur des Maskenanwenders anpassbar ist.

Die Skizze Fig.5a zeigt eine Variante des Einsatzelementes 16 nach welcher deren gelartig vernetzter Körper eine hufeisenartige Gestalt aufweist und sich in Applikationsposition über den Nasenrücken des Maskenanwenders erstreckt.

Die Skizze Fig.5b zeigt eine Variante des Einsatzelementes 16 nach welcher deren gelartig vernetzte Körper ringartig der Dichtlippe folgend, um eine Mund- und/oder Nasenöffnung umlaufend ausgebildet ist.

Der Querschnitt des Einsatzelementes 16 im Bereich der Schnittebene π entspricht beispielsweise dem in Figur 4 dargestellten Querschnitt.

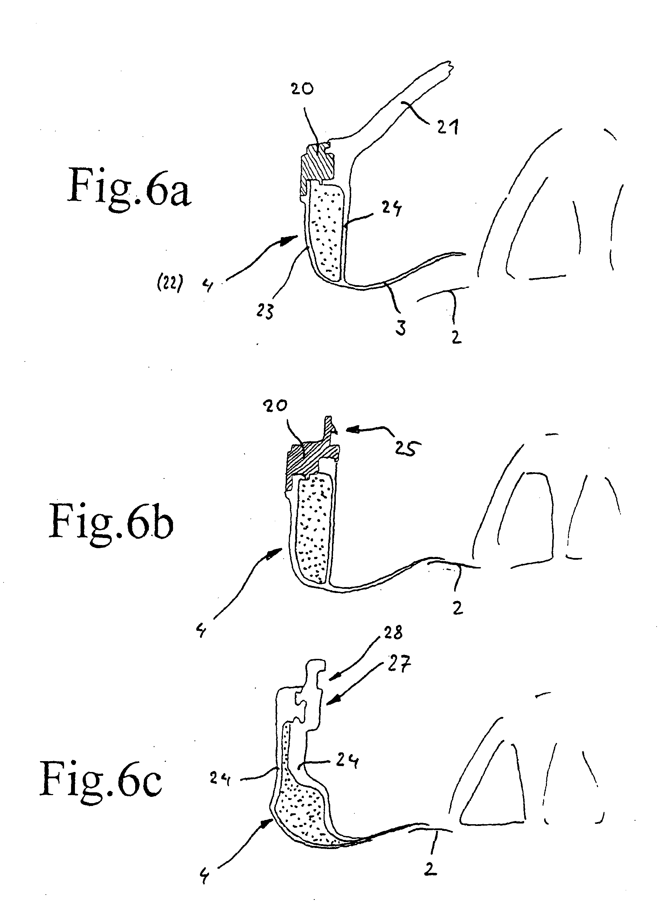

Figur 6a zeigt eine Schnittansicht durch eine Dichtkisseneinrichtung ebenfalls mit einer querschnittsverdickten und einem Gel- oder Schaummaterial ausgefüllten Zone die hier in einem integral mit der Dichtlippeneinrichtung gebildeten Taschenabschnitt 22 gebildet ist, wobei der Taschenabschnitt 22 durch ein Rahmenelement 20 abgedeckt ist und die Dichtlippeneinrichtung mit einem Schalenkörper 21 versehen ist, der integral mit der Dichtlippeneinrichtung, insbesondere der Dichtlippe 3 aus einem Elastomermaterial gefertigt ist. Der Taschenabschnitt 22 ist durch zwei Wandabschnitte 23, 24 definiert, die Teil der Dichtlippeneinrichtung bilden. Das Rahmenelement 20 bildet Teil einer Applikationsvorrichtung zur Applikation der Dichtkisseneinrichtung auf dem Gesicht eines Atemmaskenanwenders. An dem Rahmenelement sind Profilierungen und Raststrukturen vorgesehen, die eine definierte Koppelung der Dichtlippeneinrichtung mit dem Rahmenelement 20 ermöglichen. Das Rahmenelement kann aus einem biegsamen Material gefertigt sein, so dass eine weitere Anpassung des Maskenkissens an die individuelle Gesichtstektur eines Maskenanwenders ermöglicht ist. Bei dem gezeigten Ausführungsbeispiel bildet das Rahmenelement 20 eine Haltestruktur, durch welche der Taschenabschnitt 22 abgeschlossen wird. Das Rahmenelement 20 kann mit der Dichtlippeneinrichtung spaltfrei verklebt sein.

Figur 6b zeigt eine Schnittansicht durch eine Dichtkisseneinrichtung ähnlich Figur 6a, wobei das Rahmenelement 20 eine Anschlussstruktur 25 bildet, zur Ankoppelung einer den Nasenbereich überdeckenden, beispielsweise aus POM gefertigten Maskenschale, das Rahmelement 20 bildet auch bei diesem Ausführungsbeispiel eine Haltestruktur, durch welche der Taschenabschnitt 4 verschlossen ist.

Figur 6c zeigt eine weitere Schnittansicht durch eine Dichtkisseneinrichtung ebenfalls mit einer querschnittsverdickten und mit einem nachgiebigen Gel- oder Schaummaterial gefüllten querschnittsverdickten Zone 4. Die Dichtlippeneinrichtung umfasst ähnlich wie die Ausführungsform nach Figur 6a zwei Wandabschnitte 23, 24 zwischen welchen das Gel- oder Schaummaterial aufgenommen ist. Die Wandabschnitte 23, 24 weisen eine Verschlussprofilierung 27 auf und sind durch diese zusammengehalten. Die so gebildete Dichtlippeneinrichtung kann über einen Halterandabschnitt 28 an einer Hartschale einer Atemmaske angebracht werden;

In den Figuren 6a, 6b und 6c ist andeutungsweise der Nasenbereich eines Patienten dargestellt. Die Querschnittsgestaltung der Dichtlippeneinrichtung kann in ihrem Verlauf in Umfangsrichtung variieren und unterschiedliche Stützcharakteristika und Flächenpressungen erzeugen. Vorzugsweise liegt der Flächenanteil der unter Wirkung des Gel- oder Schaumstoffkörpers abgestützten Hautkontaktzone der Dichtlippeneinrichtung im Bereich von 18 bis 54 %. Der von der Gel- oder Polsterkörpergestützen Zone umsäumte Flächenanteil der Hautkontaktzone liegt vorzugsweise im Bereich von 46 bis 82%.

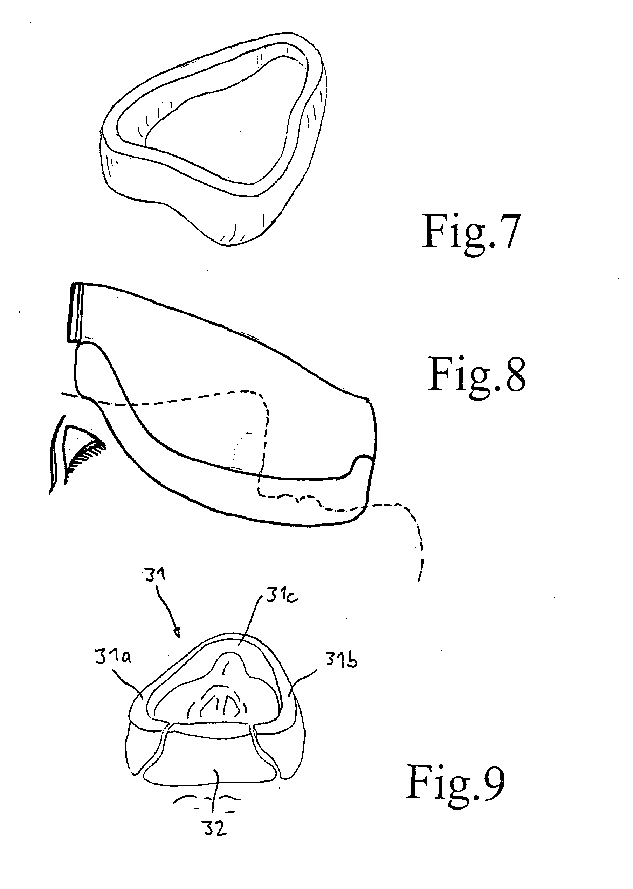

In Figur 7 ist eine vereinfachte perspektivische Darstellung eines aus einem Gel- oder Schaummaterial bestehenden profilierten Polsterkörpers zur Abstützung einer Atemmaske gezeigt, der in seinem Verlauf in Umfangsrichtung verschiedene Querschnitte bereitstellt. Der Polsterkörper ist derart ausgebildet, dass dieser im Bereich der zur Überbrückung des Nasenrückens vorgesehenen Zone nur eine relativ kleine Querschnittsfläche aufweist. In den zur Abstützung auf Backenknochen oder Wangenbereich vorgesehenen Zonen ist der Polsterkörper relativ dickwandig und stark verformbar ausgebildet.

Figur 8 zeigt eine vereinfachte Seitenansicht zur Erläuterung eines möglichen Verlaufs eines Polsterkörpers nach Figur 7 auf der Gesichtsfläche eines Maskenanwenders. Der Polsterkörper kann in einem hinreichend komplementär ausgebildeten Taschenabschnitt einer Dichtlippeneinrichtung aufgenommen sein, wie diese in Verbindung mit den Figuren 6a, 6b und 6c sowie den Figuren 14, 15, 16 beschrieben ist.

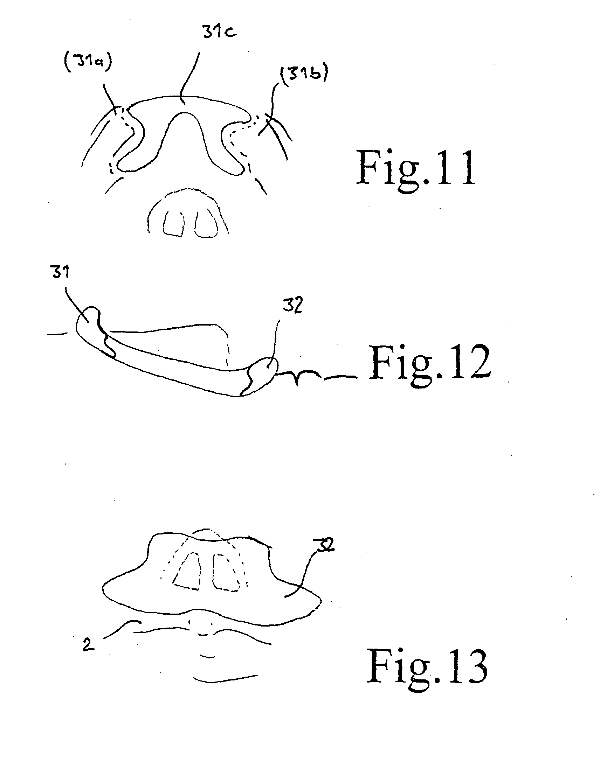

Figur 9 zeigt eine vereinfachte perspektivische Darstellung eines Gel- oder Schaumpolsterkörpers, der in verschiedene Segmente unterteilt ist, wobei die Segmente unterschiedliche mechanische Eigenschaften, insbesondere unterschiedliche Shorehärten aufweisen und die Grenzflächen organisch uneben ausgebildet sind. Bei dem gezeigten Ausführungsbeispiel ist der Polsterkörper aus einem Gelmaterial gefertigt und in ein Flankenstützsegment 31 und ein Oberlippenstützsegment 32 unterteilt. Die beiden Segmente sind aus unterschiedlich eingefärbten, transparenten Gelmaterialien gefertigt und in eingebautem Zustand in einer Umfangstasche einer Dichtlippeneinrichtung aufgenommen. Das Flankenstützsegment 31 weist einen linken Flankenabschnitt 31a und einen rechten Flankenabschnitt 31b auf, die hier im Nasenrückenbereich über eine Brückenzone 31c miteinander verbunden sind. Es ist auch möglich, im Bereich der Brückenzone ein von den Flankenabschnitten 31a, 31b separates, vorprofiliertes Segment vorzusehen, wie dies in Figur 11 dargestellt ist, es ist auch möglich, die Beiden Flankenabschnitte 31a, 31b im Beriech des Nasenrückens zu unterbrechen.

Die Segmente 31a, 31b, 31c, 32 sind vorzugsweise so ausgebildet, dass diese über uneben ausgebildete Grenzflächen aneinander stoßen. Der Verlauf der Grenzflächen kann unter ästhetischen sowie strukturmechanischen Gesichtspunkten abgestimmt werden und dabei insektenpanzerartige Segmentgrenzkonturen aufweisen.

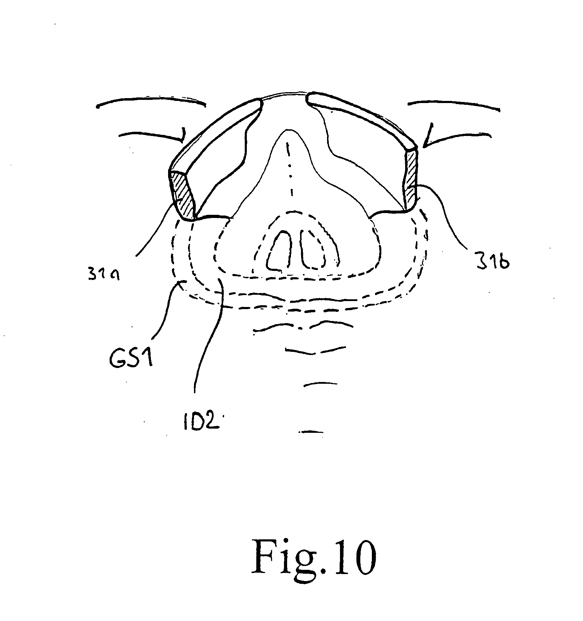

Figur 10 zeigt eine räumliche Darstellung zur Erläuterung einer Polsterkörpervariante mit einem im Nasenrückenüberbrückungsbereich ausgesparten Abschnitt. Die Flankenabschnitte 31a, 31b bilden Stützsysteme zur Übertragung einer Maskenstützkraft auf die Gel- oder Schaumkörperstützzone G/S1. Diese Gel- oder Schaumkörperstützzone G/S1 umsäumt eine Innenlippenauflagezone ID2 die sich über den Oberlippen oder Kinnbereich, sowie den Nasenrückenbereich erstreckt. Dieser Polsterkörper kann in einen Taschenabschnitt einer Dichtlippeneinrichtung eingesetzt sein.

Fig. 11 zeigt eine Schnittansicht eines den Nasenrückenbereich überbrückenden Polsterkörpersegmentes 31c das aus einem besonders weichen, schwachvernetztem Gelmaterial gefertigt ist und den Nasenrücken überquert. Das Polsterkörpersegment 31c ist so ausgebildet, dass dieses Anschlussflächen aufweist die einen mechanisch vorteilhaften Übergang zu den Seitenflankensegmenten 31a, 31b ermöglichen.

Fig. 12 zeigt eine Skizze zur Illustration des Übergangsbereiches zwischen einem Polsterkörpersegment 31c und einem Seitenflankensegment 31a, sowie insgesamte den Verlauf eines derartigen, aus mehreren Segmenten gebildeten Polsterkörpers auf der Gesichtsfläche eines Patienten. Das jeweils weichere Segment bildet einen Auslaufabschnitt der das härtere Segment untergreift.

Figur 13 zeigt eine Skizze zur Erläuterung eines auf dem Oberlippenbereich eines Patienten aufsitzenden Polsterkörpersegments 32 wie dies bei dem segmentierten Polsterkörper gemäß Figur 12 vorgesehen ist. Die Polsterkörpersegmente bestehen vorzugsweise aus einem Gel- oder Elastomermaterial mit niedrigem Vernetzungsgrad und sind als solche in komplementär ausgebildeten Taschenabschnitten unmittelbar aneinanderstoßend, oder durch Stege voneinander getrennt aufgenommen.

Figur 14 zeigt eine perspektivische Ansicht einer Dichtlippeneinrichtung einschließlich zugehörigem Maskenbasisköpers, wobei die Dichtlippeneinrichtung einen Umfangstaschenabschnitt 4 aufweist der zur Bereitstellung eines Polsterkörpers mit einem Gel- oder Schaumstoffmaterial gefüllt ist (siehe Figuren 6a, 6b nd 6c). Die derart ausgebildete Dichtlippeneinrichtung stützt im Bereich der durch Wirrschraffur abgesetzt gekennzeichneten Dichtlippenumfangszone den Maskenbasiskörper auf der Gesichtsfläche des Patienten in der Zone GS1 (siehe auch Fig. 10). Der Innenbereich der Dichtlippeneinrichtung 3 liegt von den Maskenanpresskräften weitgehend unabhängig über die Zone ID2 auf der Gesichtsfläche des Maskenanwenders auf.

Der Umfangstaschenabschnitt 4 ist von der Wandung 23 eingefasst. Die von der Wandung 23 umschlossene und im Zusammenspiel mit einer Innenumfangswandung 24 gebildete Tasche ist durch ein Rahmenelement 20 abgedeckt. Über das Rahmenelement 20 kann unmittelbar eine Aufbringung von Maskenanpresskräften auf das im Taschenabschnitt 4 aufgenommene Gel- oder Polstermaterial erfolgen.

In Figur 15 ist eine perspektivische Ansicht einer Dichtlippeneinrichtung ähnlich Figur 14 einschließlich zugehörigem Maskenbasisköpers gezeigt, wobei die Dichtlippeneinrichtung einen Umfangstaschenabschnitt aufweist der mit einem Gel- oder Schaumstoffmaterial gefüllt ist und im Bereich der abgesetzt gekennzeichneten Dichtlippenumfangszone den Maskenbasiskörper auf der Gesichtsfläche des Patienten abstützt. Im Bereich des Nasenrückens ist der Polsterkörper mit einer Ausnehmung versehen oder unterbrochen, so dass in dieser Zone keine Abstützung über das Gel- oder Schaumstoffmaterial auf dem Nasenrücken erfolgt;

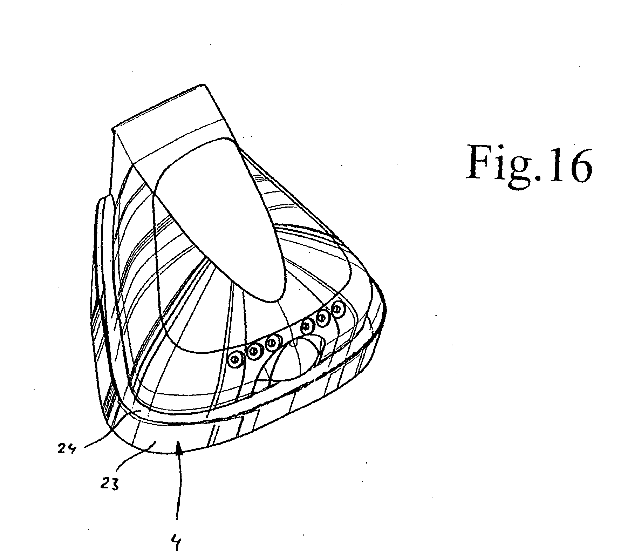

Figur 16 zeigt eine perspektivische Ansicht der Dichtlippeneinrichtung nach Figur 15 zur Illustration des Umfangstaschenabschnitts 4. In diesen Umfangstaschenabschnitt kann das zur Bildung des Polsterkörpers vorgesehene Material ungeformt eingebracht werden und in dem Taschenabschnitt definiert vernetzen. Es ist auch möglich, in den Taschenabschnitt vorgeformte Polsterkörper, insbesondere segmentierte Polsterkörper einzusetzen und darin zum Beispiel durch ein Rahmenelement zu sichern.

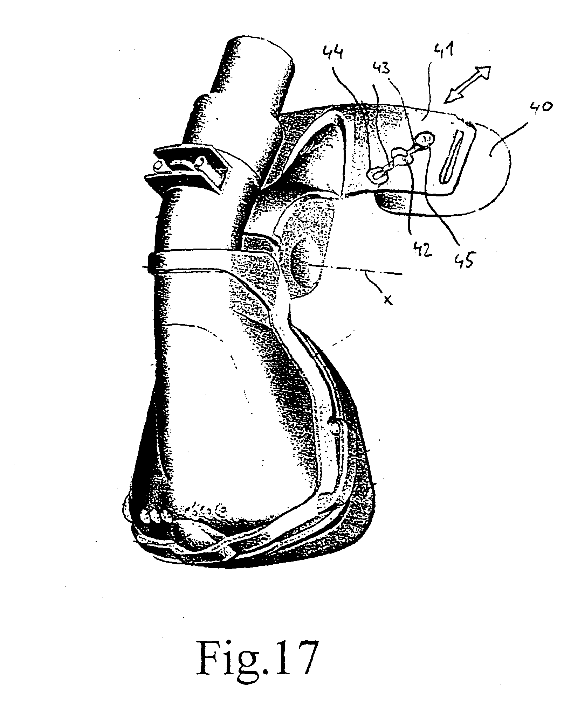

Figur 17 zeigt eine perspektivische Ansicht einer Atemmaske mit schwenkbewegbar angelenkter Stirnauflageeinrichtung und einer Dichtlippeneinrichtung einschließlich zugehörigem Maskenbasisköpers, gemäß Figuren 14 oder 15. Einzelheiten dieser Atemmaske sind in der Patentanmeldung PCT-EP02/02877 beschrieben. Der Offenbarungsgehalt dieser Patentanmeldung ist durch diese Bezugnahme in die vorliegende Anmeldung eingeschlossen. Diese Atemmaske umfasst Stirnpads 40 die aus einem Elastomermaterial gefertigt sind und an einer Stirnabstützeinrichtung 41 angebracht sind.

Die Befestigung der Stirnpads 40 erfolgt über eine Steckhalterung die eine zentrale Aufnahmeöffnung 42 und anschließende Rasbahnen 43 aufweist, die in Fixiertäler 44 einmünden. In diese Fixiertäler 44 kann ein Rastkopfabschnitt 45 des Stirnpads 40 eintauchen und das Stirnpad 40 in dieser Stellung sichern. Die Rastbahnen 43 sind so ausgelegt, dass das Stirnpad 40 in einer auf den Maskenanwender abgestimmten Position an der Stirnauflageeinrichtung 41 festgelegt werden kann.

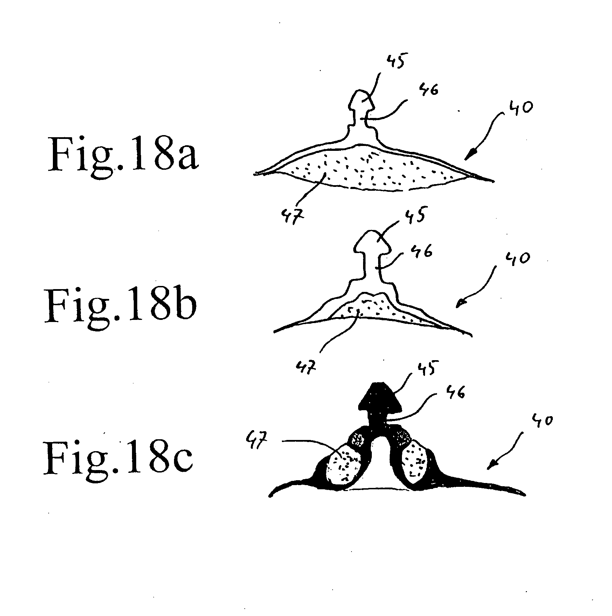

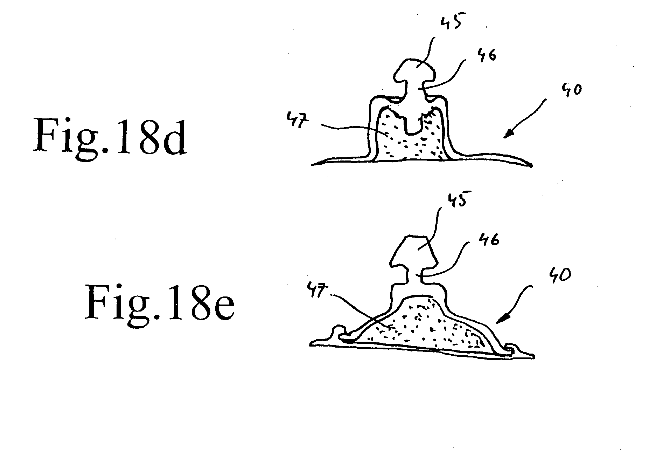

In den Figuren 18a bis 18e sind Schnittansichten von Varianten von Strinauflage-Pads 40 insbesondere zur Verwendung in Verbindung mit einer Atemmaske, beispielsweise gemäß Figur 17 dargestellt, wobei die Stirnauflagepads 40 querschnittsverdickte Zonen aufweisen die mit einem Gel- oder Schaumstoffmaterial gefüllt sind. Die Stirnauflagepads 40 umfassen einen Stützschaft 46 und eine Rastkopfabschnitt 45. Der Stützschaft 46 und der Rastkopfabschnitt 45 sind so ausgebildet, dass diese eine definierte Kippbewegung der zur Auflage auf der Stirnfläche eines Patienten vorgesehenen Auflagezone ermöglichen. Im Bereich dieser Auflagezone sind Profilierungen vorgesehen, die einem Unerdruckaufbau vorbeugen.

Die Pads 40 sind mit einem Gel- oder Schaumstoffmaterialabschnitt 47 versehen. Dieser Abschnitt 47 sowie die diesen umgrenzenden elastomeren Wandungen sind so abgestimmt, dass sich eine definierte Flächenpressungsverteilung und/oder eine definierte Gelenkcharakteristik ergibt.

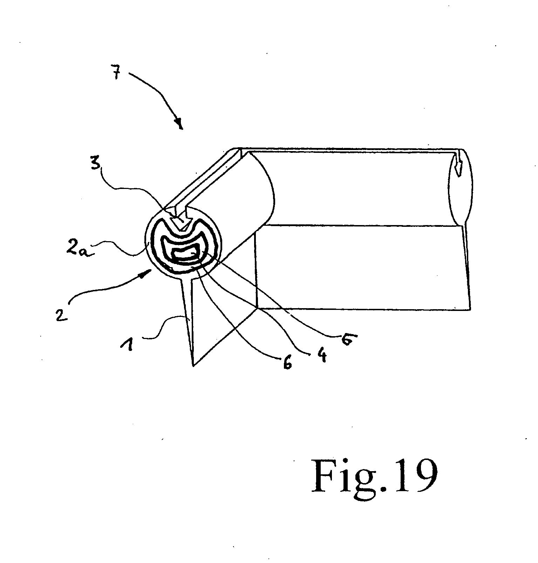

Die in Figur 19 gezeigte Dichtungsstruktur 7 umfaßt eine Dichtlippe 1 und eine sich daran anschließende querschnittsverdickte Zone 2. Im Bereich der querschnittsverdickten Zone 2 ist eine Befestigungsprofilierung 3 vorgesehen, über welche die Dichtungsstruktur an einem Halteprofil befestigbar ist.

Im Bereich der querschnittsverdickten Zone 2 ist das Profilmaterial der Dichtungsstruktur derart verarbeitet, dass die Shore-Härte des Profilmateriales im Kernbereich 4 der querschnittsverdickten Zone niedriger ist als im Randbereich 2a der querschnittsverdickten Zone. Im Kernbereich 4 hat das Profilmaterial im wesentlichen gel-artige Materialeigenschaften. In dem zwischen dem Kernbereich 4 und dem Randbereich 2a liegenden Bereichen 5, 6 ist das Profilmaterial derart vernetzt, dass sich hier zwar höhere Shore-Härten als im Kernbereich 4, jedoch niedrigere Shore-Härten als im Randbereich 2a ergeben. Die Shore-Härte in dem, dem Kernbereich 4 benachbarten Innenbereich 5 ist bei diesem Ausführungsbeispiel niedriger als die Shore-Härte in dem angrenzenden Ringbereich 6.

Die erfindungsgemäße Dichtungsstruktur zeichnet sich dadurch aus, dass diese eine teilausgehärtete, querschnittsverdickte Zone aufweist. Die erfindungsgemäße Dichtungsstruktur ist vorzugsweise aus einem additionsvernetzenden Material, insbesondere Silikonmaterial als Spritzguß- oder Extrusionsprofil gefertigt. Durch Abstimmung des Temperaturprofils einer Formraum oder Extrüsionskanalwand können die gewünschten Vernetzungsgrade in einem engen Toleranzbereich festgelegt werden. Gemäß einer besonders bevorzugten Ausführungsform der Erfindung wird die Vernetzungsreaktion im Bereich der querschnittsverdickten Zone aktiv unterbunden, so dass die gewünschten Materialeigenschaften der querschnittsverdickten Zone langzeitstabil aufrechterhalten werden können.

Die Vernetzungsreaktion kann insbesondere gestoppt werden, indem die Katalysereaktion durch Zugabe oder Aktivierung eines Katalyse-Blockes unterbunden wird. Die Aktivierung des Katalyse-Blockes kann insbesondere durch UV-Licht durch Mikrowellen, durch elektromagnetische Strahlung oder auch durch Injektion eines Katalyse-Blockes in den Bereich der querschnittsverdickten Zone herbeigeführt werden. Gemäß einer besonders bevorzugten Ausführungsform wir die erfindungsgemäße Dichtstruktur aus einem additionsvernetzenden LSR-Silikon gefertigt, wobei der Bereich der hier dargestellten Dichtlippe durchgehärtet ist, und der sich an die Dichtlippe 1 angrenzende querschnittsverdickte Bereich Zonen mit vermindertem Vernetzungsgrad, insbesondere gel-elastischen Eigenschaften aufweist. Die querschnittsverdickte Zone 2 weist in ihrem Randbereich 2a eine vollständig vernetzte Außenhaut auf, wogegen das Material im Kernbereich 4 nahezu unvernetzt ist.

## Patentansprüche

**1.** Maskenkisseneinrichtung für eine Atemmaske mit einer Aufnahmeöffnung, die in Applikationsposition der Atemmaske wenigstens mit dem Nasen- und/oder Mundöffungsbereich eines Maskenanwenders übereinkommt, und einer aus einem elastomeren Material gebildeten, um die Aufnahmeöffnung umlaufenden Dichtlippe, die in Applikationsposition auf der Gesichtsfläche des Maskenanwenders aufsitzt, **dadurch gekennzeichnet, dass** in dem Maskenkissen wenigstens ein Aufnahmetaschenabschnitt ausgebildet ist und in diesem Aufnahmetaschenabschnitt ein Polsterkörper aus einem Gel- oder Schaummaterial vorgesehen ist.

**2.** Maskenkisseneinrichtung nach Anspruch 29, **dadurch gekennzeichnet, dass** der Verlauf des Taschenabschnitts derart festgelegt ist, dass die Dichtlippeneinrichtung eine Hautkontaktzone definiert in welcher die Dichtlippeneinrichtung durch den Polsterkörper gegen die Gesichtsfläche gedrängt wird.

**3.** Maskenkisseneinrichtung nach Anspruch 1 oder 2, **dadurch gekennzeichnet, dass** der Polsterkörper in Segmente unterteilt ist.

**4.** Maskenkisseneinrichtung nach Anspruch 1, 2 oder 3, **dadurch gekennzeichnet, dass** der Aufnahmetaschenabschnitt sich zumindest teilweise entlang des Umfangs der Maskenkisseneinrichtung erstreckt.

**5.** Maskenkisseneinrichtung nach Anspruch 4, **dadurch gekennzeichnet, dass** der Umfangstaschenabschnitt partiell unterbrochen ist.

**6.** Maskenkisseneinrichtung nach einem der vorangegangenen Ansprüche, **dadurch gekennzeichnet, dass** der Polsterkörper eine Hufeisenartige Gestalt aufweist und sich in Applikationsposition vorzugweise über den Nasenrücken des Maskenanwenders erstreckt.

**7.** Maskenkisseneinrichtung nach einem der vorangegangenen Ansprüche, **dadurch gekennzeichnet, dass** der Polsterkörper ein Gel- oder Schaummaterial aufweist.

**8.** Maskenkisseneinrichtung nach einem der vorangegangenen Ansprüche, **dadurch gekennzeichnet, dass** der Polsterkörper unterschiedliche mechanische Eigenschaften, vorzugsweise im Bereich zur Überbrückung des Nasenrückens gegenüber dem Bereich zur Abstützung im Wangenbereich, aufweist, und wobei der Körper vorzugsweise in Segmente unterteilt ist.

**9.** Maskenkisseneinrichtung nach einem der vorangegangenen Ansprüche, **dadurch gekennzeichnet, dass** der Polsterkörper im Nasenrückenüberbrückungsbereich ausgespart ist.

**10.** Maskenkisseneinrichtung nach einem der vorangegangenen Ansprüche, **dadurch gekennzeichnet, dass** der Polsterkörper unterschiedliche Segmente vorzugsweise aus einem Gel- oder Elastomermaterial, vorzugsweise mit niedrigem Vernetzungsgrad, aufweist, die vorzugsweise in Taschenabschnitten unmittelbar aneinanderstoßen oder durch Stege voneinander getrennt aufgenommen sind.

**11.** Maskenkisseneinrichtung nach einem der vorangegangenen Ansprüche, **dadurch gekennzeichnet, dass** der Aufnahmetaschenabschnitt Wandabschnitte aufweist, die vorzugsweise einen Teil der Dichtlippeneinrichtung bilden.

**13.** Maskenkisseneinrichtung nach einem der vorangegangenen Ansprüche, **dadurch gekennzeichnet, dass** die Dichtungsstruktur zumindest abschnittsweise in Applikationsrichtung federnd nachgiebig aufgehängt ist.

**14.** Maskenkisseneinrichtung nach einem der vorangegangenen Ansprüche, **dadurch gekennzeichnet, dass** die Dichtlippe über eine Faltenstruktur federnd mit der Dichtungsstruktur gekoppelt ist.

**15.** Maskenkisseneinrichtung nach einem der vorangegangenen Ansprüche, **dadurch gekennzeichnet, dass** die Maskenkisseneinrichtung ein Stirnpolster umfasst.
